# EUROPEAN PATENT SPECIFICATION

(11) **EP 0 766 557 B1**
(45) Date of publication and mention of the grant of the patent: **19.12.2001**
(21) Application number: 95926309.6
(22) Date of filing: 23.06.1995
(51) Int. Cl.: A61K 31/195, A61K 47/10, A61K 47/12, A61K 47/14

(54) **TOPICAL COMPOSITIONS COMPRISING N-ACETYL-L-CYSTEINE**
TOPISCH ANZUWENDENDE ZUBEREITUNGEN ENTHALTEND N-ACETYL-L-CYSTEIN
COMPOSITIONS POUR APPLICATION LOCALE CONTENANT DE LA N-ACETYLE-L-CYSTEINE

(30) Priority: 23.06.1994 US 264549
(43) Date of publication of application: 09.04.1997
(73) Proprietor: THE PROCTER & GAMBLE COMPANY, Cincinnati, Ohio 45202 (US)
(72) Inventor: HILLEBRAND, Greg, George, Fairfield, OH 45014 (US); SCHNICKER, Marcia, Sterling, Hamilton, OH 45011 (US)
(74) Representative: McKinney, Victoria
(86) International application number: US9509024
(87) International publication number: WO9600060

(56) References cited:
- WO-A-91/07090
- WO-A-93/04669
- WO-A-95/00136
- FR-A- 2 698 545
- CHEMICAL ABSTRACTS, vol. 121, no. 12, 19 September 1994, Columbus, Ohio, US; abstract no. 141216, & JP,A,06 157 257 (KAO CORP.,JP) 3 June 1994
- CHEMICAL ABSTRACTS, vol. 120, no. 10, 7 March 1994, Columbus, Ohio, US; abstract no. 116497, & JP,A,05 294 812 (KAO CORP.,JP) 9 November 1993

## Description

### TECHNICAL FIELD

The present invention relates to topical compositions having N-acetyl-L-cysteine as an active constituent, more particularly such compositions that have improved efficacy and stability.

### BACKGROUND OF THE INVENTION

N-acetyl-L-cysteine (hereinafter alternatively referred to as "NAC") has a number of applications in the fields of therapeutics and cosmetics. For example, sterile solutions of NAC, given orally or intravenously, will lessen the toxic effects of acetaminophen overdosage. Sterile solutions of NAC, given orally or by inhalation, are also widely used as mucolytics. NAC has also been formulated in oils, lotions, milks, ointments, creams, gels, sprays, etc. NAC is useful topically for a variety of uses including the treatment of acne, inflammation of the skin, eczema, sunburn and regulating skin wrinkles. Compositions containing NAC have been described, for example, in International Publication No. WO 94/14428, published on July 1, 1994; and U.S. Patent No. 5,411,991, issued May 2, 1995, Shander and Ahluwalia; International Publication No. WO 93/10755, published June 10, 1993, Blank et al.; and in U.S. Patent No. 5,296,500, Hillebrand, issued on March 22, 1994, and in WO 93/04669.

For many topical indications, NAC is formulated and packaged for multiple dosing. This usually will require that the NAC formulation be preserved against microbial contamination. For this reason, preservatives are frequently added to the NAC formulation. For example, preservatives that have been described for use in NAC compositions are those commercially available under the trade names GERMALL®, GERMALL 115®, and GLYDANT®, (DMDM hydantoin, diazolidinyl urea and imidazolidinyl urea, respectively).

Three basic criteria should be met by a preservative system so as to more effectively preserve an NAC formulation for topical use. First, the preservative should retain efficacy in the composition against microorganisms for a reasonable period of time if the product is to be sold commercially (i.e., the composition must have an acceptable shelf life or stability). (Such efficacy being alternatively referred to herein as preservative efficacy). This preservative efficacy is important not only to ensure an esthetically appealing product, but also to ensure the activity of the active components of the formulation. Second, the preservative (along with other components of the composition) should not cause any undesirable reactions (e.g., significant skin irritation or sensitization) in the user population. Finally, the preservative (along with other components of the composition) should not react with or cause the degradation of any of the formula constituents, particularly the active components.

We have now found that certain agents may decrease the activity of the NAC in topical formulations. First, an excessive number of microorganisms may decrease the activity of the NAC, for example by microbial metabolism of the NAC. Second, it has been found that formaldehyde chemically reacts with NAC to decrease its activity. Thus, when a composition containing NAC is formulated with a formaldehyde or a formaldehyde forming preservative or other material, the composition may have decreased activity over time relative to the corresponding formulation that does not contain formaldehyde or a compound capable of forming formaldehyde. Therefore, it would be desirable to provide NAC compositions that have preservative efficacy and which do not include formaldehyde or formaldehyde forming preservatives or other materials.

While the art has provided various compositions containing NAC, it has not heretofore addressed the need for compositions containing NAC and a preservative system that meets each of the aforementioned criteria of preservative efficacy, absence of undesirable user reactions, and stability of the NAC active. Accordingly, none has provided NAC compositions in the manner or to the extent of the present invention.

It is an object of the present invention to provide compositions comprising NAC which have preservative efficacy to provide a suitable shelf life (shelf stability) of the composition.

It is a further object to provide compositions comprising NAC which have preservative efficacy to provide a suitable shelf life of the NAC.

It is a further object of this invention to provide topical compositions comprising NAC which do not cause undesirable reactions, e.g., significant skin irritation including drying, or skin sensitization, in the user population.

It is a further object of this invention to provide topical formulations comprising NAC which contain no ingredients which react with or degrade NAC so as to decrease its efficacy. Thus, it is an object of the invention to provide compositions comprising NAC having extended NAC chemical stability as compared to existing compositions.

### SUMMARY OF THE INVENTION

The present invention relates to topical compositions comprising an active comprising NAC, a preservative, and a cosmetically acceptable and/or pharmaceutically - acceptable carrier. The compositions are substantially free of formaldehyde and materials that may form or release formaldehyde when present in the composition, including preservatives that may form or release formaldehyde in the composition. Formaldehyde and materials that may form or release formaldehyde in the composition are hereinafter alternatively referred to as "formaldehyde donor(s)."

The preservative is selected from benzyl alcohol, propylparaben, ethylparaben, butylparaben, methylparaben, benzylparaben, isobutylparaben, phenoxyethanol, ethanol, sorbic acid, benzoic acid, methylchloroisothiazolinone, methylisothiazolinone, methyl dibromoglutaronitrile, dehydroacetic acid, o-phenylphenol, phenol, sodium bisulfite, dichlorophen; and mixtures and salts of any of the foregoing.

The compositions have a pH of less than 7 and are preferably substantially free of panthenol and carraghenate. Preferred compositions also contain only limited amounts, if any, of materials that may cause reactions in the user population, e.g., monovalent alcohols.

### DETAILED DESCRIPTION

As used herein, the term "topical composition" means a composition that is suitable for directly laying or spreading on outer skin.

As used herein, "substantially free of formaldehyde donors" means that there are no no detectable formaldehyde donors, preferably no formaldehyde donors. The presence of formaldehyde donors may be indicated by the presence of formaldehyde in the composition by any suitable analytical technique, for example high pressure liquid chromatography. The presence of such donors may be detected initially or evidenced by the generation of formaldehyde over time.

As used herein, the term "active" means an agent that is in the composition to provide a benefit to the user of the composition, e.g., skin benefit

As used herein, the term "preservative" means a material that prevents the growth and or reacts with and/or destroys microorganisms that might damage or grow on the product or otherwise contaminate it.

As used herein, the term "cosmetically acceptable and/or pharmaceutically-acceptable" means that salts, drugs, medicaments or inert ingredients which the term describes are suitable for use in contact with the tissues of humans and lower animals without undue toxicity, incompatibility, instability, irritation, allergic response, and the like, commensurate with a reasonable benefit/risk ratio.

As used herein, the term "substantially free of panthenol" means that the composition comprises less than 0.08% by weight of panthenol.

As used herein, the term "substantially free of carraghenate" means that the composition comprises less than 0.15% by weight of carraghenate.

As used herein, the term "safe and effective amount" means an amount of compound or composition sufficient to significantly induce a positive modifcation in the condition to be treated, but low enough to avoid serious side effects (at a reasonable benefit/risk ratio), within the scope of sound medical judgment. As may be applicable to certain uses of the present compositions, the safe and effective amount of the compound or composition will vary with the particular condition being treated, the age and physical condition of the patient being treated, the severity of the condition, the duration of the treatment, the nature of concurrent therapy, the specific compound or composition employed, the particular pharmaceutically-acceptable carrier utilized, and like factors within the knowledge and expertise of an attending physician.

As used herein, all percentages are by weight unless otherwise specified.

The topical compositions of the invention comprise as an active a safe and effective amount of NAC. The NAC provides a variety of skin treating utilities such as are known in the art, including but not limited to the treatment of acne, inflammation of the skin, eczema, sunburn and regulating skin wrinkles.

As used herein, NAC includes N-acetyl-L-cysteine itself and derivatives thereof. Derivatives of N-acetyl-L-cysteine include cosmetically- and/or pharmaceutically- acceptable salts thereof, including, but not limited to alkali metal salts, e.g., sodium, lithium, potassium and rubidium salts; alkaline earth metal salts, e.g, magnesium, calcium and strontium salts; non-toxic heavy metal salts, e.g., aluminum salts and zinc salts; boron salts; silicon salts; ammonium salts; trialkylammonium salts, e.g., trimethylammonium and triethylammonium; and tetralkylonium salts. Preferred cosmetically- and/or pharmaceutically- acceptable salts of N-acetyl-L-cysteine include Na⁺, K⁺, Ca⁺⁺, Mg⁺⁺, Al₂(OH)₅⁺, NH₄⁺, (HOCH₂CH₂)₃NH⁺, (CH₃CH₂)₃NH⁺, (C₃CH₂)₄N⁺, C₁₂H₂₅(CH₃)₃N⁺ and C₁₂H₂₅(C₅H₄N)₃N⁺ salts. More preferred salts of N-acetyl-L-cysteine include Na⁺, K⁺, NH₄⁺, and (HOCH₂CH₂)₃NH⁺ salts. Most preferred salts of N-acetyl-L-cysteine include Na⁺ and NH₄⁺ salts. Suitable salts of N-acetyl-L-cysteine are described, for example, in U.S. Patent No. 5,296,500, issued to Hillebrand on March 22, 1994. Mixtures including N-acetyl-L-cysteine and/or derivatives thereof are suitable for use herein.

Active compounds having the structures shown and described in the above referenced U.S. Patent No. 5,296,500 are also suitable for use in this invention, in addition to N-acetyl-L-cysteine or a derivative thereof. Cosmetically- and/or pharmaceutically- acceptable salts of those active compounds are also suitable for use herein, in addition to N-acetyl-L-cysteine or a derivative thereof. Preferred such salts include alkali metal salts, alkaline earth metal salts, non-toxic heavy metal salts, boron salts, silicon salts, ammonium salts, trialkylammonium salts, and tetralkylonium salts such as those noted above in reference to salts of N-acetyl-L-cysteine. Suitable salts of these actives are described, for example, in the above referenced U.S. Patent No. 5,296,500. Mixtures of any of these active compounds and/or derivatives thereof are suitable for use herein. The active is N-acetyl-L-cysteine or a derivative thereof

Compositions of this invention preferably comprise from 0.005% to 25%, more preferably from 0.1% to about 15%, still more preferably from 0.5% to 10%, yet more preferably from 1% to 7%, even more preferably from 2% to 5%, most preferably 2% of NAC.

In addition to NAC, the compositions of the present invention may comprise one or more other actives, including but not limited to sunscreens, sunblocks, antiinflammatory agents, moisturizers, antioxidants and radical scavengers. Several actives of these types are known in the art and are suitable for use herein.. Preferred other actives are those which do not significantly reduce the activity of the NAC. In a preferred embodiment, the active consists essentially of NAC.

The topical compositions of the invention also comprise one or more preservatives. Suitable preservatives are those which are substantially free of formaldehyde donors. Thus, the preservatives useful herein are those that do not form or release formaldehyde in the composition either in the process of preserving or in an unrelated process. In contrast to the preservatives suitable for the present invention, formaldehyde forming or releasing preservatives form or release formaldehyde in the composition either in the process of preserving or in an unrelated process. Examples of formaldehyde forming or releasing preservatives include DMDM hydantoin, diazolidinyl urea, imidazolidinyl urea (the foregoing preservatives being commercially available, for example, as GLYDANT®, GERMALL®, and GERMALL 115®, respectively), formaldehyde itself, and the like.

The preservative is selected from benzyl alcohol, propylparaben, ethylparaben, butylparaben, methylparaben, benzylparaben, isobutylparaben, phenoxyethanol, ethanol, sorbic acid, benzoic acid, methylchloroisothiazolinone, methylisothiazolinone (a preservative containing a mixture of methylchloroisothiazolinone and methylisothiazolinone being commercially available, for example, from Rohm & Haas as Kathon CG®), methyl dibromoglutaronitrile (commercially available, for example, from Calgon as Tektamer 38®), dehydroacetic acid, o-phenylphenol, phenol, sodium bisulfite, dichlorophen, salts of any of the foregoing compounds, and mixtures of any of the foregoing compounds.

More preferred preservatives are selected from the group consisting of benzyl alcohol, propylparaben, methylparaben, phenoxyethanol, methylchloroisothiazolinone, methylisothiazolinone, benzoic acid, salts of any of the foregoing preservatives, and mixtures of any of the foregoing compounds.

Even more preferred preservatives are benzyl alcohol, propylparaben, methylparaben, phenoxyethanol and mixtures thereof. Still more preferably, the preservative is a mixture of propylparaben and methyl paraben with either or both of benzyl alcohol and phenoxyethanol. In addition to NAC stability, these mixtures provide broad preservative efficacy with no or only minimal risk of skin irritation to the user. Most preferably, the preservative is a mixture of benzyl alcohol, propylparaben and methylparaben. In addition to NAC stability and broad preservative efficacy, this mixture presents a particularly low risk of skin irritation to the user.

The level of each preservative when present is as follows:
(1) from 0.002% to 2% of benzoic acid, preferably from 0.02% to 1%, more preferably from 0.01% to 0.5% benzoic acid. For example, a preferred composition comprises 0.2% of benzoic acid.
(2) from 0.01% to 10% of benzyl alcohol, preferably from 0.1% to 5%, more preferably from 0.2% to 3%, most preferably from 0.2% to 1% benzyl alcohol. For example, a preferred composition comprises 0.5% of benzyl alcohol. Compositions containing benzyl alcohol in an amount of less than 3% are preferred as presenting a lower risk of reactions in the user, with amounts of less than 1% being more preferred as presenting an even lower risk of such reactions.
(3) from 0.0015% to 2% of benzylparaben, butylparaben and/or dehydroacetic acid, preferably from 0.015% to 0.6%, more preferably, from 0.05% to 0.5%, of benzylparaben, butylparaben and/or dehydroacetic acid. For example, a preferred composition comprises 0.15% benzylparaben, butylparaben and/or dehydroacetic acid.
(4) from 0.004% to 4% dichlorophen, preferably from 0.04% to 2%, more preferably from 0.02% to 1% dichlorophen. For example, a preferred composition comprises 0.4% dichlorophen.
(5) from 0.01% to 50% ethanol, preferably from 0.1% to 30%, more preferably from 1% to 20% ethanol. For example, a preferred composition comprises 10% ethanol. At lower levels, e.g., less than 15%, the ethanol may function less as a preservative and more as a preservative enhancer such as described herein.
(6) from 0.0015% to 2% of ethylparaben and/or isobutylparaben, preferably from 0.015% to 0.5%, more preferably from 0.05% to 0.4% ethylparaben and/or isobutylparaben. For example, a preferred composition comprises 0.15% ethylparaben and/or isobutylparaben.
(7) from 0.001% to 2% of Kathon CG®, preferably from 0.01% to 0.5%, more preferably from 0.05% to 0.4% Kathon CG®. For example, a preferred composition comprises 0.1% Kathon CG®.
(8) from 0.003% to 3% of methylparaben, preferably from 0.03% to 1%, more preferably from 0.03% to 0.5% methylparaben. For example, a preferred composition comprises 0.3% methylparaben.
(9) from 0.0014% to 10% o-phenylphenol and/or phenol, preferably from 0.014% to 5%, more preferably from 0.14% to 3% o-phenylphenol and/or phenol. For example, a preferred composition comprises 1.4% o-phenylphenol and/or phenol.
(10) from 0.01% to 10% phenoxyethanol, preferably from 0.1% to 5%, more preferably from 0.2% to 2% phenoxyethanol, most preferably from 0.75 to 1.0%. For example, a preferred composition comprises 0.5% phenoxyethanol.
(11) from 0.002% to 2% propylparaben and/or sodium bisulfite, preferably from 0.02% to 1%, more preferably from 0.1% to 0.5% propylparaben and/or sodium bisulfite. For example, a preferred composition comprises 0.2% propylparaben and/or sodium bisulfite.
(12) from 0.0005% to 1% sorbic acid, preferably from 0.005% to 0.5%, more preferably from 0.025% to 0.1% sorbic acid. For example, a preferred composition comprises 0.05% sorbic acid.
(13) from 0.0006% to 1% Tektamer 38®, preferably from 0.006% to 0.5%, more preferably from 0.03% to 0.1% Tektamer 38®. For example, a preferred composition comprises 0.06% Tektamer 38®.

In more preferred embodiments of the present invention, the composition may comprise from 0.01% to 10% benzyl alcohol and/or 0.01% to 10% phenoxyethanol with 0.002% to 2% propylparaben and 0.003% to 3% methyl paraben. In even more preferred embodiments, the composition comprises from 0.1% to 5% benzyl alcohol and/or 0.1% to 5% phenoxyethanol with 0.02% to 1% propylparaben and 0.03% to 1% methyl paraben. Still more preferred embodiments include from 0.2% to 3% benzyl alcohol and/or 0.2% to 2% phenoxyethanol with 0.1% to 0.5% propylparaben and 0.03% to 0.5% methyl paraben. In the most preferred embodiments, the composition comprises from 0.2% to 1% benzyl alcohol and/or 0.75 to 1.0% phenoxyethanol, 0.1% to 0.5% propylparaben and 0.03% to 0.5% methylparaben. In regard to the foregoing percentage compositions, it is further preferred that the preservative be a mixture of benzyl alcohol, methyl paraben, and propyl paraben.

The compositions of this invention also include a cosmetically acceptable and/or pharmaceutically - acceptable carrier (alternatively referred to herein as "carrier") to enable the NAC and optional other actives to be delivered to the desired target (e.g., skin) at an appropriate concentration. The carrier can thus act as a diluent, dispersant, or solvent for the active(s) which ensures that it can be applied to and distributed evenly over the selected target at an appropriate concentration. The carrier may be solid, semi-solid or liquid. The carrier can itself be inert or it can possess physiological or pharmaceutical benefits of its own.

The selection of a carrier presents a wide range of possibilities depending on the required product form of the composition. Carriers useful in compositions of this invention can include water, one or more cosmetically and/or pharmaceutically-acceptable materials other than water, or mixtures thereof Generally, the carrier is either aqueous or organic in nature or an aqueous emulsion, and is capable of having the NAC dispersed or dissolved therein. Organic carriers are exemplified by lower monovalent alcohols (e.g., C₁ - C₄) and low molecular weight glycols and polyols. Other cosmetically- and/or pharmaceutically-acceptable materials include emollients, skin penetration enhancing agents, coloring agents, fragrances, emulsifiers, thickening agents, and solvents, e.g., capable of dissolving one or more of the active(s). Such other cosmetically- and/or pharmaceutically-acceptable materials are known in the art. For example, such materials are described in Harry's Cosmeticology, 7th Ed., Harry & Wilkinson (Hill Publishers, London 1982); in Pharmaceutical Dosage Forms- Disperse Systems; Lieberman, Rieger & Banker, Vols. 1 (1988) & 2 (1989); Marcel Decker, Inc.; in The Chemistry and Manufacture of Cosmetics, 2nd. Ed., deNavarre (Van Nostrand 1962-1965); and in The Handbook of Cosmetic Science and Technology, 1st Ed.. Knowlton & Pearce (Elsevier 1993).

As used herein, "emollient" refers to a material used for the prevention or relief of dryness, as well as for the protection of the skin. A wide variety of suitable emollients are known and may be used herein. Such emollients include, but are not limited to, hydrocarbon oils and waxes, silicon oils, triglyceride fats and oils, acetoglyceride esters, ethoxylated glycerides, alkyl esters of fatty acids having 10 to 20 carbon atoms, alkenyl esters of fatty acids having 10 to 20 carbon atoms, fatty acids having 8-22 carbon atoms, fatty alcohols having 8-22 carbon atoms, fatty alcohol ethers, ether-esters, lanolin and derivatives, polyhydric alcohols and their polyether derivatives, wax esters, beeswax derivatives, vegetable waxes, phospholipids, sterols, and amides. SAGARIN, COSMETICS, SCIENCE AND TECHNOLOGY, 2nd Edition, Vol. 1, pp. 32-43 (1972), incorporated herein by reference, contains numerous examples of suitable emollient materials. Compositions of this invention typically comprise from 1% to 50%, preferably from 5% to 20% of an emollient.

The carrier is preferably one which can aid and/or enhance penetration into the skin. Carriers useful in the topical compositions according to the invention may include penetration enhancing agents such as are known in the art, including liposomes, latex lattices, microspheres, cyclodextrans and various forms of microencapsulation of the active. A preferred amount of penetration enhancing agent is from 1% to 5% of the composition.

Preferred carriers for use in this invention comprise a safe and effective amount of a preservative enhancer. As used herein, the term "preservative enhancer" means an agent whose purpose is to enhance the activity of the preservative. As will be understood by the artisan having ordinary skill, the preservative enhancer does not itself typically provide sufficient efficacy; it tends to increase the efficacy of the preservative. Enhancement of the preservative efficacy may involve chelation. Preferred preservative enhancers useful in the present invention include ethylenediaminetetraacetic acid (EDTA), butylene glycol, propylene glycol, ethanol, and mixtures thereof.

In alternatively preferred embodiments, topical compositions of the present invention comprise:
(1) from 0.001% to 5% EDTA, more preferably from 0.01% to 1%, more preferably still from 0.05% to 0.5% EDTA. For example, a preferred composition comprises about 0.1% EDTA.
(2) from 0.01% to 1% of butylene glycol, more preferably from
   0.1% to 5%, more preferably still from 0.5% to 3% butylene glycol. For example, a preferred composition comprises 1.5% butylene glycol.
(3) from 0.01% to 50% propylene glycol, more preferably from 0.1% to 20%, more preferably still from 1% to 15% propylene glycol. For example, a preferred composition comprises 10% propylene glycol.
(4) as a preservative enhancer, from 0.01% to less than 15% ethanol. For example, a preferred composition comprises 10% ethanoL

Where the preservative includes a paraben, e.g., methyl or propyl paraben, EDTA is the preferred preservative enhancer and is preferably used at the above levels.

The compositions of the invention preferably contain zinc or a zinc salt which may complex with the NAC. Without being bound by theory, the zinc most likely removes odor by complexing with malodorous H₂S which may be formed in trace amounts as the NAC decomposes. The zinc may additionally or alternatively increase the stability of the sulfhydryl compound. The use of zinc salts in a manner which is suitable for the present invention is further described in the above referenced U.S. Patent No. 5,296,500.

The compositions of the present invention are formulated to have a pH of below 7. The pH values of these compositions preferably range from 2 to 6, more preferably from 3 to 6, most preferably from 4.5 to 5.5. Compositions having a pH within the range of 4.5 to 7 tend to exhibit less skin irritation, less odor, and greater shelf stability relative to corresponding compositions having a pH of greater than 8.5.

Preferred compositions of this invention, in addition to being substantially free of formaldehyde and formaldehyde forming components, are substantially free of panthenol and substantially free of carraghenate.

For topical application, the compositions of this invention are preferably in the form of a lotion, solution, ointment, serum, spray, tonic, cream, bar, cream rinse, gel, stick, mousse, paste, milk and the like. These forms are well known in the art and are described, for example, in the aforementioned references regarding known cosmetically- and/or pharmaceutically-acceptable materials.

Thus, the topical compositions of the present invention can be formulated as liquids, for example as a lotion, mousse, solution or milk. Such liquid compositions may be formulated for use in conjunction with an applicator such as a roll-ball applicator, a pad applicator, or a spray device such as an aerosol can containing propellant, or a container fitted with a pump to dispense the liquid product, or a liquid-impregnated fabric, such as a tissue wipe. Alternatively, the compositions of the invention can be solid or semi-solid, for example sticks, serums, creams or gels. Such solid or semi-solid compositions may be formulated for use in conjunction with a suitable applicator or simply a tube, jar or other convenient container.

Preferred lotions of this invention comprise a safe and effective amount of the NAC; from 1% to 50%, preferably from 3% to 15% of an emollient; and from 45% to 85%, preferably from 50% to 75% water. Optionally, the lotion form may contain a suitable emulsifier, comprising from 3% to 50%, preferably from 10% to 20% of the composition. Emulsifiers may be nonionic, anionic or cationic. Suitable emulsifiers are disclosed in, for example, U.S. Patent No. 3,755,560, issued August 28, 1973, Dickert et al.; U.S. Patent No. 4,421,769, issued December 20, 1983, Dixon et al.; and McCutcheon's Detergents and Emulsifiers, North American Edition, pp. 317-324 (1986). Preferred emulsifiers are anionic or nonionic.

Preferred solutions of the present invention comprise a safe and effective amount of the NAC, water and a suitable organic solvent. Suitable organic materials useful as the solvent or a part of a solvent system include water soluble or water dispersible hydroxy compounds, organic acids or esters of such hydroxy compounds or acids, such as: propylene glycol, glycerin, polyethylene glycol (e.g., molecular weight of from about 200-600), polypropylene glycol (e.g., molecular weight of from 425-2025), sorbitol esters, 1,2,6-hexanetriol, ethanol, isopropanol, diethyl tartrate, butanediol, and mixtures thereof

Stick-form compositions of the invention preferably comprise a safe and effective amount of NAC, and from 50% to 98%, preferably from 60% to 90%, of one or more of the previously described emollients. Such compositions can optionally further comprise from 1% to 20%, preferably from 5% to 15%, of a suitable thickening agent, one or more emulsifiers and/or water.

Gel compositions of the present invention can be formulated by simply mixing a suitable thickening agent to the previously described solution compositions. The gel compositions preferably comprise a safe and effective amount of NAC; from 5% to 75%, preferably from 10% to 50%, of an organic solvent as previously described for solutions; and from 0.5% to 20%, preferably from 1% to 10% of the thickening agent.

Preferred creams of the present invention comprise a safe and effective amount of NAC; from 5% to 50%, preferably from 10% to 25%, of an emollient; and from 25% to 95% water. Optionally, the cream form contains one or more suitable emulsifiers. When an emulsifier is included, it is in the composition at a level from 3% to 50%, preferably from 5% to 20%. Examples of suitable and preferred emulsifiers are included herein above in the disclosure of lotion formulations.

The preferred lotions, solutions, sticks, gels, and creams more preferably also contain a preservative enhancer, zinc, and/or a zinc salt as previously described. These agents may be incorporated into the aforementioned formulations in the amounts previously described.

The compositions of the present invention are generally prepared by conventional methods such as are known in the art of making topical compositions. Such methods typically involve mixing of the ingredients to a relatively uniform state, ith or without heating, cooling, application of vacuum, and the like.

For optimum stability of the NAC, the compositions of this invention should be manufactured, packaged and stored in a manner which avoids simple air oxidation of the NAC. Thus, exposure of the compositions to air during manufacture, packaging and storage should be minimized.

The compositions of this invention are topically applied to the skin of a mammal, including humans and other animals, in need of treatment. The compositions are applicable to a variety of uses including the treatment of acne, inflammation of the skin, eczema, sunburn and regulating skin wrinkles and hair growth.

### Examples

The following nonlimiting examples are provided to illustrate the preparation of topical compositions in accordance with this invention. The scope of the invention is to be determined by the claims which follow. All parts, percentages, and ratios used herein are by weight unless otherwise specified.

### Reference Example I

Prepare a topical composition in solution form by combining the following components utilizing conventional mixing techniques and adjusting the pH to about 6.0 with sodium hydroxide.

| Component | % by weight |
|---|---|
| NAC | 5.0 |
| Propylene glycol | 25.0 |
| Ethanol | 50.0 |
| Sodium Hydroxide | 1.5 |
| Water | 18.5 |

### Example II

Prepare a topical composition in solution form by combining the following components utilizing conventional mixing techniques and adjusting the pH to about 4.5 with sodium hydroxide.

| Component | % by weight |
|---|---|
| NAC | 2.0 |
| Propylene glycol | 1.5 |
| Ethanol | 20.0 |
| Water | 69.1 |
| Benzyl alcohol | 2.0 |
| Glycerin | 3.0 |
| Myristyl alcohol | 2.0 |
| Sodium hydroxide | 0.4 |

### Example III

Prepare a topical composition in solution form by combining the following components utilizing conventional mixing techniques and adjusting the pH to about 3.0 with sodium hydroxide.

| Component | % by weight |
|---|---|
| NAC | 1.0 |
| Propylene glycol | 30.0 |
| Glycerin | 3.0 |
| Sodium hydroxide | 0.1 |
| Methyl paraben | 0.25 |
| Water | 65.65 |

### Reference Example IV

Prepare a topical composition in solution form by combining the following components utilizing conventional mixing techniques and adjusting the pH to about 5.0 with sodium hydroxide.

| Component | % by weight |
|---|---|
| NAC | 0.5 |
| Propylene glycol | 30.0 |
| Propylene glycol laurate | 1.0 |
| Isopropanol | 20.0 |
| Sodium hydroxide | 0.1 |
| Water | 48.4 |

### Example V

Prepare a lotion by combining the following components utilizing conventional mixing techniques and adjusting the pH to about 4.0 with sodium hydroxide.

| Component | % by weight |
|---|---|
| NAC | 5.0 |
| Di-partially hydrogenated tallow | |
| dimethyl ammonium chloride | 4.0 |
| Cetyltrimethyl ammonium chloride | 2.0 |
| DC-200 fluid (12500 csk)* | 1.0 |
| Citric acid | 3.5 |
| Ethylene glycol distearate | 1.5 |
| PEG-3 C₁₂ alkyl amide | 3.0 |
| Sodium hydroxide | 1.0 |
| Phenoxyethanol | 0.5 |
| Methylparaben | 0.25 |
| Propylparaben | 0.1 |
| EDTA | 0.1 |
| Water | 78.05 |

| | |
|---|---|
| *Dimethylpolysiloxane available from by Dow Chemical Co. | |

### Examples VI - VIII

Prepare lotions by combining the following components using conventional mixing techniques and adjusting the pH to about 4.5 with sodium hydroxide.

| Component | VI | VII | VIII |
|---|---|---|---|
| | % by weight | % by weight | % by weight |
| NAC | 0.1 | 0.5 | 2.0 |
| Hydroxyethyl cellulose | 0.4 | --- | 0.4 |
| Absolute ethanol | 15.0 | 15.0 | 15.0 |
| Propane-1,2-diol | --- | --- | 30.6 |
| Butane-1,3-diol | 33.4 | 33.4 | --- |
| Sodium benzoate | 0.2 | 0.2 | 0.2 |
| Perfume | 0.5 | 0.5 | 0.5 |
| Sodium hydroxide | 0.02 | 0.1 | 0.4 |
| Water | 50.38 | 50.3 | 50.9 |

### Example IX

Prepare a water-in-oil emulsion by combining the following ingredients using conventional mixing techniques and adjusting the pH to about 6.5 with sodium hydroxide.

| | Component | % by weight |
|---|---|---|
| Oil Phase | | |
| | Cetearyl alcohol | 5.0 |
| | Silicon oil, 200 fluid | 1.0 |
| | Isopropyl myristate | 2.0 |
| | Sodium stearoyl-2-lactylate | 2.0 |
| | Propylparaben | 0.1 |
| Aqueous Phase | | |
| | NAC | 5.0 |
| | Propylene glycol | 5.0 |
| | Sodium citrate | 0.2 |
| | Perfume | 0.1 |
| | Methylparaben | 0.25 |
| | EDTA | 0.1 |
| | Sodium hydroxide | 1.0 |
| | Water | 78.25 |

### Example X

Prepare an oil-in-water cream by mixing the following components and adjusting the pH to about 3.5 with sodium hydroxide. Prepare the cream by first separately (1) mixing the oil phase and heating to 65°C and (2) combining the aqueous phase and heating to 70°C; then adding the aqueous phase to the oil phase with suitable agitation; then cooling the mixture to room temperature. Apply moderate agitation while cooling.

| | Component | % by weight |
|---|---|---|
| Oil Phase | | |
| | Sorbitan monoleate | 20.0 |
| | Liquid paraffin | 60.0 |
| Aqueous Phase | | |
| | NAC | 5.0 |
| | Butylene glycol | 1.5 |
| | Xanthan gum | 1.0 |
| | Phenoxyethanol | 0.5 |
| | Perfume | 0.2 |
| | Sodium hydroxide | 0.8 |
| | Water | 11.0 |

### Example XI

Prepare an oil-in-water cream by mixing the following components and adjusting the pH to 4.5. Prepare the cream as described for Example X.

| | Component | % by weight |
|---|---|---|
| Oil Phase | | |
| | Perfume | 0.20 |
| | Cetyl alcohol, NF | 1.00 |
| | Stearyl alcohol, NF | 1.00 |
| | Polyoxyethylene (50:50 - 12/20) cetyl/stear (50:50) | 1.00 |
| | Propylene glycol dicaprylate/dicaprate | 3.00 |
| | Glycerol monostearate | 2.00 |
| | Glyceryl monostearate-palmitate | 2.00 |
| Water Phase | | |
| | N-acetyl-L-cysteine | 5.25 |
| | Distilled Water | 77.19 |
| | Glycerin | 3.00 |
| | Citric acid | 0.50 |
| | Benzyl alcohol | 0.50 |
| | Propylparaben | 0.1 |
| | Methylparaben, NF | 0.25 |
| | Zinc oxide, USP | 0.26 |
| | Butylene glycol | 1.50 |
| | Sodium hydroxide | 1.12 |
| | disodium EDTA | 0.13 |

The cream exhibits enhanced shelf stability, particularly of the NAC, relative to a corresponding composition which contains a formaldehyde donor such as a preservative which forms or releases formaldehyde in the composition as part of the preservation or another process. Thus, the cream exhibits enhanced NAC efficacy, relative to the same corresponding composition.

## Claims

1. A topical composition, **characterized in that** it comprises:
(a) an active comprising N-acetyl-L-cysteine or a derivative thereof, and
(b) a preservative selected from the group consisting of:
benzoic acid in an amount of from 0.002% to 2%;
benzyl alcohol in an amount of from 0.01% to 10%;
benzylparaben, butylparaben and/or dehydroacetic acid in an amount of from 0.0015% to 2%;
dichlorophen in an amount of from 0.004% to 4%;
ethylparaben and/or isobutylparaben in an amount of from 0.0015% to 2%; methylchloroisothiazolinone and/or methylisothiazolinone in an amount of from 0.001% to 2%;
methylparaben in an amount of from 0.003% to 3%;
o-phenylphenol and/or phenol in an amount of from 0.0014% to 10%;
phenoxyethanol in an amount of from 0.01% to 10%;
propylparaben and/or sodium bisulfite in an amount of from 0.002% to 2%; sorbic acid in an amount of from 0.0005% to 1%;
methyl dibromoglutaronitrile in an amount of from 0.0006% to 1%;
salts of any of the foregoing compounds; and
mixtures of any of the foregoing;
(c) a cosmetically and/or pharmaceutically acceptable carrier;
the composition further **characterized by** being substantially free of formaldehyde donors
the composition further **characterized by** having a pH of less than 7.

2. A topical composition according to Claim 1 wherein the preservative is selected from benzoic acid; benzyl alcohol; methylchloroisothiazolinone; methylisothiazolinone; methylparaben; phenoxyethanol; propylparaben; salts of any of the foregoing compounds; and mixtures of any of the foregoing.

3. A topical comparition according to Claim 1 or 2 wherein the preservative is selected from benzyl alcohol, propylparaben, methylparaben, phenoxyethanol, and mixtures thereof.

4. A topical composition, according to any of Claims 1 to 3 wherein the preservative comprises a mixture of (i) methyl paraben; (ii) propyl paraben; and (iii) benzyl alcohol and/or phenoxyethanol.

5. A topical composition according to any of Claims 1 to 4 wherein the comparition is substantially free of panthenol and carraghenate.

6. The composition of any of Claims 1 to 5, wherein said preservative comprises benzyl alcohol, methyl paraben and propyl paraben.

7. The composition of Claim 6 wherein said preservative consists essentially of benzyl alcohol, propylparaben and methylparaben.

8. The composition of any of Claims 1 to 5 wherein said preservative comprises phenoxyethanol, methyl paraben and propyl paraben.

9. The composition of Claim 8 wherein said preservative consists essentially of phenoxyethanol, propylparaben and methylparaben.

10. The composition of any of the preceding Claims wherein the composition comprises from 0.005% to 25% N-acetyl-L-cysteine or a derivative thereof.

11. The composition of any of the preceding Claims further comprising a preservative enhancer, preferably ethylenediaminetetraacetic acid.

12. The composition of any of the preceding Claims further comprising an agent selected from the group consisting of emollients, skin penetration agents, zinc, and zinc salts.

## Patentansprüche

1. Topische Zusammensetzung, **dadurch gekennzeichnet, daß** sie
(a) ein wirksames, N-Acetyl-L-cystein oder ein Derivat hievon umfassendes Mittel; und
(b) ein Konservierungsmittel, welches von der Gruppe, bestehend aus:
Benzoesäure in einer Menge von 0,002% bis 2%;
Benzylalkohol in einer Menge von 0,01% bis 10%;
Benzylparaben, Butylparaben und/oder Dehydroessigsäure in einer Menge von 0,0015% bis 2%;
Dichlorophen in einer Menge von 0,004% bis 4%;
Ethylparaben und/oder Isobutylparaben in einer Menge von 0,0015% bis 2%;
Methylchlorisothiazolinon und/oder Methylisothiazolinon in einer Menge von 0,001% bis 2%;
Methylparaben in einer Menge von 0,003% bis 3%;
o-Phenylphenol und/oder Phenol in einer Menge von 0,0014% bis 10%;
Phenoxyethanol in einer Menge von 0,01% bis 10%;
Propylparaben und/oder Natriumbisulfit in einer Menge von 0,002% bis 2%;
Sorbinsäure in einer Menge von 0,0005% bis 1%;
Methyldibromglutaronitril in einer Menge von 0,0006% bis 1%;
Salze von jedweden der vorstehenden Verbindungen; und Gemische von jedweden der vorstehenden Verbindungen; und
(c) einen kosmetisch und/oder pharmazeutisch annehmbaren Träger
umfaßt, wobei die Zusammensetzung ferner **dadurch gekennzeichnet ist, daß** sie von Formaldehyddonatoren im wesentlichen frei, und die Zusammensetzung ferner **dadurch gekennzeichnet ist, daß** sie einen pH-Wert von weniger als 7 besitzt.

2. Topische Zusammensetzung nach Anspruch 1, wobei das Konservierungsmittel unter Benzoesäure, Benzylalkohol, Methylchlorisothiazolinon, Methylisothiazolinon, Methylparaben, Phenoxyethanol, Propylparaben; Salzen von jedweden der vorstehenden Verbindungen und Gemischen von jedweden der vorstehenden Verbindungen ausgewählt ist.

3. Topische Zusammensetzung nach Anspruch 1 oder 2, wobei das Konservierungsmittel unter Benzylalkohol, Propylparaben, Methylparaben, Phenoxyethanol und Gemischen hievon ausgewählt ist.

4. Topische Zusammensetzung nach einem der Ansprüche 1 bis 3, wobei das Konservierungsmittel ein Gemisch aus (i) Methylparaben; (ii) Propylparaben; und (iii) Benzylalkohol und/oder Phenoxyethanol umfaßt.

5. Topische Zusammensetzung nach einem der Ansprüche 1 bis 4, wobei die Zusammensetzung von Panthenol und Carraghenat im wesentlichen frei ist.

6. Zusammensetzung nach einem der Ansprüche 1 bis 5, wobei das genannte Konservierungsmittel Benzylalkohol, Methylparaben und Propylparaben umfaßt.

7. Zusammensetzung nach Anspruch 6, wobei das genannte Konservierungsmittel im wesentlichen aus Benzylalkohol, Propylparaben und Methylparaben besteht.

8. Zusammensetzung nach einem der Ansprüche 1 bis 5, wobei das genannte Konservierungsmittel Phenoxyethanol, Methylparaben und Propylparaben umfaßt.

9. Zusammensetzung nach Anspruch 8, wobei das genannte Konservierungsmittel im wesentlichen aus Phenoxyethanol, Propylparaben und Methylparaben besteht.

10. Zusammensetzung nach einem der vorstehenden Ansprüche, wobei die Zusammensetzung 0,005% bis 25% N-Acetyl-L-cystein oder eines Derivates hievon umfaßt.

11. Zusammensetzung nach einem der vorstehenden Ansprüche, welche ferner ein Verstärkungsmittel für das Konservierungsmittel umfaßt, vorzugsweise Ethylendiamintetraessigsäure.

12. Zusammensetzung nach einem der vorstehenden Ansprüche, welche ferner ein Mittel umfaßt, welches von der aus Emollientien, Mitteln für die Hautpenetration, Zink und Zinksalzen bestehenden Gruppe ausgewählt ist.

## Revendications

1. Composition pour application topique, **caractérisée en ce qu'**elle comprend :
(a) un ingrédient actif comprenant de la N-acétyl-L-cystéine ou un dérivé de celle-ci ; et
(b) un conservateur sélectionné parmi le groupe composé de :
l'acide benzoïque dans une quantité comprise entre 0,002 % et 2 % ;
l'alcool benzylique dans une quantité comprise entre 0,01 % et 10 % ;
le benzylparabène, le butylparabène et/ou l'acide déhydro-acétique dans une quantité comprise entre 0,0015 % et 2 % ;
le dichlorophéne dans une quantité comprise entre 0,004 % et 4 % ;
l'éthylparabène et/ou l'isobutylparabène dans une quantité comprise entre 0,0015 % et 2 % ;
la méthylchloroisothiazolinone et/ou la méthylisothiazolinone dans une quantité comprise entre 0,001 % et 2 % ;
le méthylparabène dans une quantité comprise entre 0,003 % et 3 % ;
le o-phénylphénol et/ou le phénol dans une quantité comprise entre 0,0014 % et 10 % ;
le phénoxyéthanol dans une quantité comprise entre 0,01 % et 10 % ;
le propylparabène et/ou le bisulfite de sodium dans une quantité comprise entre 0,002 % et 2% ;
l'acide sorbique dans une quantité comprise entre 0,0005 % et 1 % ;
le méthyldibromoglutaronitrile dans une quantité comprise entre 0,0006 % et 1% ;
les sels de l'un quelconque des composés précédents ; et
des mélanges d'un nombre quelconque des composés précédents ;
(c) un agent véhiculeur acceptable d'un point de vue cosmétique et/ou pharmaceutique ;
la composition étant en outre **caractérisée en ce qu'**elle est substantiellement exempte de donneurs de formaldéhyde,
la composition étant en outre **caractérisée en ce qu'**elle possède un pH inférieur à 7.

2. Composition pour application topique selon la revendication 1, dans laquelle le conservateur est sélectionné parmi l'acide benzoïque ; l'alcool benzylique; la méthylchloroisothiazolinone ; la méthylisothiazolinone ; le méthylparabène ; le phénoxyéthanol ; le propylparabène ; des sels de l'un quelconque des composés précédents ; et des mélanges d'un nombre quelconque des composés précédents.

3. Composition pour application topique selon la revendication 1 ou 2, dans laquelle le conservateur est sélectionné parmi l'alcool benzylique, le propylparabène, le méthylparabène, le phénoxyéthanol et des mélanges de ceux-ci.

4. Composition pour application topique selon l'une quelconque des revendications 1 à 3, dans laquelle le conservateur comprend un mélange de (i) méthylparabène ; (ii) propylparabène; et (iii) alcool benzylique et/ou phénoxyéthanol.

5. Composition pour application topique selon l'une quelconque des revendications 1 à 4, dans laquelle la composition est substantiellement exempte de panthénol et de carraghénate.

6. Composition selon l'une quelconque des revendications 1 à 5, dans laquelle ledit conservateur comprend de l'alcool benzylique, du méthylparabène et du propylparabène.

7. La composition de la revendication 6, dans laquelle ledit conservateur se compose essentiellement d'alcool benzylique, de propylparabène et de méthylparabène.

8. La composition selon l'une quelconque des revendications 1 à 5, dans laquelle ledit conservateur comprend du phénoxyéthanol, du méthylparabène et du propylparabène.

9. La composition selon la revendication 8, dans laquelle ledit conservateur se compose essentiellement du phénoxyéthanol, du propylparabène et du méthylparabène.

10. La composition selon l'une quelconque des revendications précédentes, dans laquelle la composition comprend 0,005 % à 25 % de N-acétyl-L-cystéine ou d'un dérivé de celle-ci.

11. La composition selon l'une quelconque des revendications précédentes, qui comprend en outre un renforçateur de conservateur, de préférence de l'acide éthylènediaminetétraacétique.

12. La composition selon l'une quelconque des revendications précédentes, qui comprend en outre un agent sélectionné parmi le groupe composé d'émollients, d'agents de pénétration dans la peau, de zinc et de sels de zinc.
